Europäisches Patentamt

European Patent Office

Office européen des brevets

(19)

(11) Numéro de publication : **0 669 302 A1**

## (12) DEMANDE DE BREVET EUROPEEN

(21) Numéro de dépôt : **95400125.1**

(51) Int. Cl.$^6$ : **C07C 17/38,** C07C 19/08

(22) Date de dépôt : **23.01.95**

(30) Priorité : **24.02.94 FR 9402114**

(43) Date de publication de la demande :
**30.08.95 Bulletin 95/35**

(84) Etats contractants désignés :
**BE DE ES FR GB GR IT NL**

(71) Demandeur : **ELF ATOCHEM S.A.**
**4 & 8, Cours Michelet**
**La Défense 10**
**F-92800 Puteaux (FR)**

(72) Inventeur : **Bertocchio, René**
**49 bis, Rue des vallières**
**F-69390 Vourles par Vernaison (FR)**
Inventeur : **Lacues, Philippe**
**46, Rue des Gobins**
**F-69390 Charly (FR)**
Inventeur : **Lantz, André**
**Domaine de la Hêtraie**
**F-69390 Vernaison (FR)**

(74) Mandataire : **Leboulenger, Jean et al**
**Elf Atochem S.A.**
**Département Propriété Industrielle, Cedex 42 -**
**La Défense 10**
**F-92091 Paris la Défense (FR)**

(54) **Procédé de purification du pentafluoroéthane.**

(57)     L'invention concerne la purification du pentafluoroéthane (F125) contenant du chloropentafluoroéthane (F115).
    Le mélange F125-F115 à purifier est soumis à une distillation extractive, l'agent d'extraction étant un alcane ou un cycloalcane en $C_5$ à $C_8$.

EP 0 669 302 A1

L'invention concerne la purification du pentafluoroéthane (F125) contenant du chloropentafluoroéthane (F115) et a plus particulièrement pour objet un procédé de purification dans lequel le F115 est éliminé par distillation extractive et facilement récupéré en vue de sa transformation ultérieure en produits inoffensifs pour l'atmosphère terrestre.

Le pentafluoroéthane est l'un des substituts possibles des chlorofluorocarbures (CFC) qui sont concernés par le protocole de Montréal et se caractérisent par une durée de vie exceptionnellement élevée leur permettant d'atteindre les hautes couches de l'atmosphère et de participer ainsi sous l'influence du rayonnement UV à la destruction de la couche d'ozone. Il est donc évident que leurs substituts ne devront, en fonction des divers procédés d'obtention, ne renfermer que des traces de ces CFC.

Les substituts sont généralement obtenus soit par des méthodes de fluoruration appropriées qui ne sont pas hautement sélectives et peuvent générer par dismutation des composés perhalogénés du type CFC, soit à partir de CFC eux-mêmes par des méthodes de réduction, en pratique par des réactions d'hydrogénolyse. C'est ainsi que le pentafluoroéthane (F125) peut être préparé par fluoration du perchloroéthylène ou de ses produits de fluoration intermédiaires tels que le dichlorotrifluoroéthane (F123) et le chlorotétrafluoroéthane (F124), ou par hydrogénolyse du chloropentafluoroéthane (F115). Dans les deux cas, le F125 produit contient des quantités non négligeables de F115 qu'il convient, le F115 étant un CFC, d'éliminer le plus complètement possible.

Or, l'existence d'un azéotrope F115/F125 à 21 % en poids de F115 (voir le brevet US 3 505 233) avec un point d'ébullition (-48,5°C sous 1,013 bar) très voisin de celui du F125 (-48,1°C) rend pratiquement impossible la séparation complète du F115 et du F125 par distillation. L'élimination du F115 dans le F125 ne peut donc se faire que par voie chimique ou par des méthodes physiques mettant en jeu un tiers corps.

Dans la demande de brevet EP 508 631 qui décrit la production d'hydrofluorocarbures (HFC) par réduction chimique en phase liquide de composés chlorés, bromés ou iodés avec un hydrure métallique ou un complexe d'un tel hydrure, il est indiqué que ce procédé peut être intéressant pour purifier certains HFC comme le F125. Dans le même but, la demande de brevet japonais publiée (Kokai) sous le n°2001414/90 utilise des couples rédox métalliques en milieu solvant. D'autres techniques comme celle décrite dans Journal of Fluorine Chemistry, 1991 vol. 55, p.105-107, utilisent des réducteurs organiques tels que le formiate d'ammonium en milieu DMF et en présence de persulfate d'ammonium.

Ces procédés qui font appel à des réactifs difficiles à manipuler (hydrures métalliques) ou susceptibles de poser des problèmes d'effluents, sont peu compatibles avec une production industrielle de F125 en tonnages importants.

Pour une fabrication industrielle de F125, la technique de distillation extractive apparaît être le procédé idéal pour éliminer le F115 résiduel.

Dans un procédé de distillation extractive, la séparation des constituants d'un mélange binaire se fait à l'aide d'une colonne dite d'extraction comportant successivement, du bouilleur à la tête, trois tronçons, l'un d'épuisement, le second d'absorption et le troisième de récupération.

Le mélange binaire à fractionner est injecté en tête du tronçon d'épuisement alors que le tiers corps jouant le rôle de solvant sélectif est introduit en tête du tronçon d'absorption de manière à circuler à l'état liquide de son point d'introduction jusqu'au bouilleur.

Le troisième tronçon dit de récupération sert à séparer par distillation le constituant le moins absorbé, des traces de solvant entraînées sous l'effet de sa tension de vapeur non nulle.

L'application de cette technique à la purification du 1,1,1,2-tétrafluoroéthane (F134a) a fait l'objet du brevet US 5 200 431 ; l'agent d'extraction utilisé est un solvant chloré ou un hydrocarbure aliphatique.

L'application de la distillation extractive à la purification du F125 est déjà décrite dans le brevet US 5 087 329 qui utilise comme agent d'extraction un hydrocarbure fluoré en $C_1$ à $C_4$ contenant éventuellement des atomes d'hydrogène et/ou de chlore et ayant un point d'ébullition compris entre -39 et +50°C. D'après les données de ce brevet, les dichlorotétrafluoroéthanes (F114 et F114a) sont au moins trois fois plus efficaces que les autres composés cités. D'autre part, 5 des 8 solvants cités sont des CFC concernés par le protocole de Montréal et dont la commercialisation devrait cesser dans un proche avenir.

L'utilisation industrielle du procédé selon ce brevet ne peut donc être économiquement envisagée que lorsque l'agent d'extraction mis en oeuvre fait partie de la chaîne d'intermédiaires conduisant au F125, c'est-à-dire en fait dans les procédés de préparation du F125 par hydrogénolyse.

Dans le cas de fabrications du F125 par fluoruration du perchloroéthylène ou de ses produits de fluoration partielle (F122, F123, F124), le brevet US 5 087 329 ne laisse le choix qu'entre des CFC que l'on ne trouvera plus sur le marché et des produits moins performants tels que le F124 ou le F123.

Il a maintenant été trouvé que les hydrocarbures saturés en $C_5$ à $C_8$ présentent des sélectivités très supérieures à celles des chlorofluoroéthanes. Le F125 est peu soluble dans ces hydrocarbures tandis que la solubilité du F115 dans ces mêmes hydrocarbures est, à poids égal de solvant, comparable à celle qu'il a dans

2

le F114 ou le F114a.

La présente invention a donc pour objet un procédé de purification d'un pentafluoroéthane contenant du chloro-pentafluoroéthane par distillation extractive, caractérisé en ce que l'on utilise comme agent d'extraction un alcane, linéaire ou ramifié, ou un cycloalcane en $C_5$ à $C_8$

Parmi ces alcanes et cycloalcanes, on préfère utiliser ceux ayant un point d'ébullition compris entre 25 et 85°C tels que, par exemple, le n- pentane, l'isopentane, le cyclopentane, le n-hexane, l'isohexane et le cyclohexane.

Le procédé selon l'invention peut être mis en oeuvre selon les principes bien connus de la distillation extractive. L'opération est effectuée dans une colonne de distillation extractive dans laquelle le mélange F125-F115 à séparer est injecté en un point situé en tête du tronçon d'épuisement. L'agent d'extraction, à savoir l'alcane ou le cycloalcane, est introduit dans la colonne en un point situé en tête du tronçon d'absorption ; il circule à l'état liquide de son point d'introduction jusqu'au bouilleur.

Le diamètre et le nombre d'étages de la colonne de distillation extractive, le taux de reflux et les températures et pressions optimales peuvent être facilement calculées par l'homme du métier à partir des données propres aux constituants individuels et à leurs mélanges (volatilités relatives, pressions de vapeur et constantes physiques).

Les exemples suivants dans lesquels les pressions indiquées sont en bars absolus illustrent l'invention sans la limiter.

L'aptitude d'un solvant à être utilisé dans la séparation par distillation extractive d'un mélange F115-F125 est appréciée au travers de sa sélectivité (S) définie comme le rapport des solubilités du F115 ($s_{115}$) et du F125 ($s_{125}$) dans le solvant à la pression partielle de 1,4 bar et à une température de 25°C:

$$S = \frac{s_{115}}{s_{125}}$$

Pour déterminer ces solubilités, on utilise un autoclave en acier inox d'une capacité de 471 ml dans lequel on introduit par piégeage ou coulée directe une quantité connue de solvant. Après avoir éliminé l'air, l'ensemble est réchauffé de la température de l'azote liquide à la température ambiante, puis placé dans une enceinte thermostatée à 25°C. On introduit alors par l'intermédiaire d'une vanne la quantité de F115 ou de F125 nécessaire pour obtenir une variation de pression voisine de 1,4 bar. Après équilibrage, on note la pression totale et on prélève par des dispositifs appropriés un échantillon de la phase liquide et un échantillon de la phase gazeuse qui sont ensuite analysés par chromatographie en phase gazeuse.

La composition molaire de la phase gazeuse permet de calculer la pression partielle du F115 ou du F125. La solubilité du F115 ou du F125 dans le solvant, exprimée en g de F115 ou de F125 par litre de solvant en phase liquide, est calculée à partir de la composition molaire de la phase liquide par la relation:

$$s = \frac{1000.x.M.d'}{x'.M'}$$

dans laquelle x et x' désignent respectivement les fractions molaires de F115 (ou de F125) et de solvant présentes dans la phase liquide (x + x' = 1), M et M' désignent respectivement la masse molaire du F115 (ou du F125) et celle du solvant, et d' est la densité du solvant en phase liquide à 25°C.

## EXEMPLE 1 (Comparatif)

On a placé dans la cellule de mesure 80,2 g de F114 et on a introduit 17,2 g de F115. Après équilibrage du système, la pression totale s'est établie à 3,25 bars et l'analyse des phases liquide et gazeuse a donné les résultats suivants :

|  | % MOLAIRE | |
|---|---|---|
|  | phase gaz | phase liquide |
| F115 | 43,1 | 15,9 |
| F114 | 56,9 | 84,1 |

soit une solubilité ($s_{115}$) de 250,9 g de F115 par litre de F114 liquide à 25°C et sous une pression partielle de 1,4 bar de F115.

On a répété l'opération avec du F125 en introduisant 6,7 g de F125 dans 82,1 g de F114 dans un premier essai, puis 10,5 g de F125 dans 80,7 g de F114 dans un second essai. Les pressions d'équilibre étant respectivement égales à 3,25 et 3,85 bars, on a observé les compositions suivantes pour les phases gaz et liquide des deux systèmes.

| | % MOLAIRE | | | |
|---|---|---|---|---|
| | Pression totale 3,25 bars | | Pression totale 3,85 bars | |
| | gaz | liquide | gaz | liquide |
| F125 | 36,4 | 6,9 | 47 | 10,7 |
| F114 | 63,6 | 93,1 | 53 | 89,3 |

soit des solubilités respectives de 76,2 g/l et de 123,3 g/l aux pressions partielles de 1,18 et 1,81 bars et une solubilité ($s_{125}$) estimée de 93 g/l sous 1,4 bar. La sélectivité définie par le rapport des solubilités du F115 et du F125 sous cette même pression est donc égale à 2,72.

## EXEMPLE 2

Dans l'appareillage décrit ci-dessus on a placé 61,6 g de n-pentane et on a introduit 13,4 g de F115. Après équilibrage, la pression s'est stabilisée à 2,05 bars et l'analyse a donné les résultats suivants.

| | % MOLAIRE | |
|---|---|---|
| | phase gaz | phase liquide |
| F115 | 68,5 | 7 |
| n-pentane | 31,5 | 93 |

Ces résultats conduisent à une solubilité ($s_{115}$) de 101,1 g/l pour une pression partielle de 1,4 bar.

On a répété l'opération en remplaçant le F115 par 5,3 g de F125 que l'on a introduit dans 58,5 g de n-pentane. Après stabilisation de la pression à 2,05 bars, la composition des deux phases était la suivante :

| | % MOLAIRE | |
|---|---|---|
| | phase gaz | phase liquide |
| F125 | 67,3 | 2,65 |
| n-pentane | 32,7 | 97,35 |

soit une solubilité ($s_{125}$) de 28,4 g/l pour une pression partielle de 1,38 bar de F125 et une sélectivité de 3,57.

## EXEMPLE 3

On a répété les opérations décrites dans l'exemple 1 en prenant successivement comme solvant le n-hexane, l'isopentane, le cyclopentane, l'isohexane, le cyclohexane et, à titre comparatif, le F124, le F113 et le tétrachlorure de carbone. Les résultats résumés dans le tableau suivant montrent que, dans le cas des alcanes et cycloalcanes, la sélectivité est très supérieure à celle obtenue avec le F114 (exemple 1 comparatif) ou d'autres solvants (F124, F113, $CCl_4$).

| SOLVANT | Solubilité à 25°C et sous une pression partielle de 1,4 bar abs. exprimée en g de F115 ou F125 par litre de solvant | | |
| --- | --- | --- | --- |
| | Solubilité du F115 g/l | Solubilité du F125 g/l | Sélectivité |
| n-hexane | 73,9 | 21,7 | 3,41 |
| isopentane | 103,7 | 31,1 | 3,34 |
| c-pentane | 64,5 | 17,8 | 3,62 |
| isohexane | 85,4 | 25,1 | 3,40 |
| c-hexane | 45,1 | 12,8 | 3,52 |
| F124 | 211 | 144 | 1,47 |
| F113 | 175 | 64 | 2,74 |
| CCl$_4$ | 59,5 | 21 | 2,83 |

## EXEMPLE 4

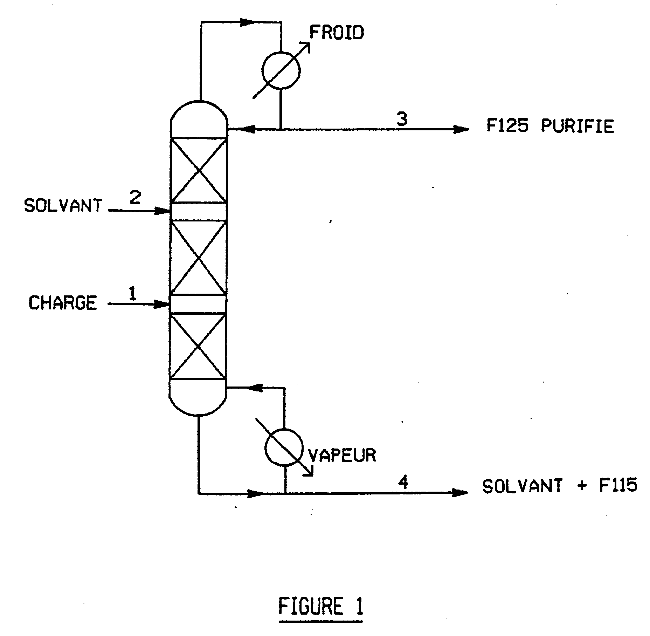

Pour séparer à l'aide de n -pentane un mélange contenant en poids 96,2 % de F125 et 3,8 % de F115, on utilise une colonne de distillation extractive de 45 plateaux théoriques représentée sur la Figure 1.

Le n-pentane est introduit au débit de 350 kg/heure par la conduite (2) au 11ème plateau théorique et le mélange F125-F115 à séparer est alimenté au débit de 170 kg/heure par la conduite (1) sur le 30ème plateau théorique. On sort en tête de la colonne de distillation extractive le F125 purifié par la conduite (3) et en pied par la conduite (4) le n-pentane enrichi en F115.

La colonne fonctionne sous une pression de 10,5 bars absolus et avec un taux de reflux égal à 6,2. Les résultats obtenus sont rassemblés dans le tableau suivant.

| COMPOSITION (en poids) | Charge 1 | Solvant 2 | Tête 3 | Pied 4 |
| --- | --- | --- | --- | --- |
| F125 | 96,2 % | - | 99,99 % | 0,1 % |
| F115 | 3,8 % | - | 125 ppm | 1,8 % |
| n-pentane | - | 100 % | 20 ppm | 98,1 % |

## EXEMPLE 5

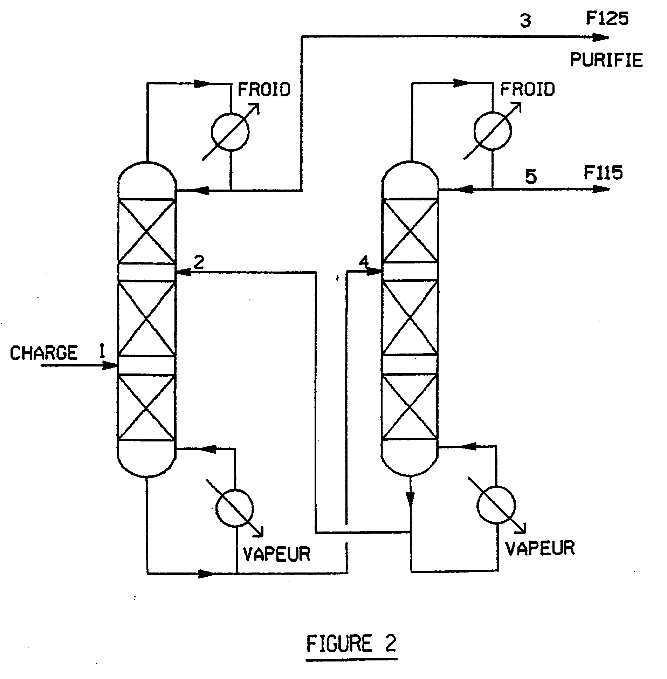

Pour séparer au moyen de cyclohexane le même mélange F125-F115 qu'à l'exemple 4, on utilise le dispositif représenté sur la Figure 2 qui combine la colonne de distillation extractive telle que décrite dans l'exemple 4 avec une colonne de distillation simple permettant le recyclage du cyclohexane.

Le F125 purifié sort en tête de la colonne de distillation extractive par la conduite (3). Le cyclohexane enrichi en F115 sortant en pied est envoyé par la conduite (4) dans la colonne de distillation simple; le F115 est récupéré en tête par la conduite (5) et le cyclohexane purifié est recyclé au 11ème plateau théorique de la colonne de distillation extractive par la conduite (2).

Avec une colonne de distillation extractive fonctionnant à 10,5 bars absolus et à un taux de reflux de 6,2, alimentée au plateau 30 avec 170 kg/heure de mélange F125-F115 et au plateau 11 avec 440 kg/heure de cyclohexane, on obtient les résultats rassemblés dans le tableau suivant.

| COMPOSITION (en poids) | Charge 1 | Solvant 2 | Tête 3 | Pied 4 |
| --- | --- | --- | --- | --- |
| F125 | 96,2 % | - | > 99,99 % | 0,1 % |
| F115 | 3,8 % | - | 11 ppm | 1,4 % |
| cyclohexane | - | 100 % | 5 ppm | 98,5 % |

**Revendications**

1.  Procédé de purification d'un pentafluoroéthane (F125) contenant du chloro-pentafluoroéthane (F115) par distillation extractive, caractérisé en ce que l'on utilise comme agent d'extraction un alcane, linéaire ou ramifié, ou un cycloalcane en $C_5$ à $C_8$.

2.  Procédé selon la revendication 1, dans lequel l'agent d'extraction est un alcane ou cycloalcane à point d'ébullition compris entre 25 et 85°C.

3.  Procédé selon la revendication 1, dans lequel l'agent d'extraction est choisi parmi le n-pentane, l'isopentane, le cyclopentane, le n-hexane, l'isohexane et le cyclohexane.

FROID

F125 PURIFIE

SOLVANT

CHARGE

VAPEUR

SOLVANT + F115

FIGURE 1

FIGURE 2

Office européen
des brevets

# RAPPORT DE RECHERCHE EUROPEENNE

Numero de la demande

EP 95 40 0125

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| P,X | EP-A-0 626 362 (SHOWA DENKO KABUSHIKI KAISHA) * le document en entier * | 1-3 | C07C17/38 C07C19/08 |
| A | US-A-3 819 493 (G.B. FOZZARD) * le document en entier * | 1-3 | |
| D,A | EP-A-0 472 391 (IMPERIAL CHEMICAL INDUSTRIES PLC.) * revendications * | 1-3 | |
| D,A | US-A-5 087 329 (V.M. FELIX) * le document en entier * | 1-3 | |

DOMAINES TECHNIQUES RECHERCHES (Int.Cl.6)

C07C

Le présent rapport a été établi pour toutes les revendications

| Lieu de la recherche | Date d'achèvement de la recherche | Examinateur |
|---|---|---|
| LA HAYE | 22 Mai 1995 | Bonnevalle, E |